# EUROPEAN PATENT APPLICATION

(11) **EP 4 785 812 A1**
(43) Date of publication of application: **05.08.2026**
(21) Application number: 24872209.2
(22) Date of filing: 24.09.2024
(51) Int. Cl.: A23L 33/135, A23L 33/21, A23L 33/125, A23L 33/175, A61K 31/198, A61K 35/745, A61K 47/30, A61K 47/36, A61P 9/00, A61P 9/10, A61P 13/12

(54) **COMPOSITION FOR IMPROVING VASCULAR ENDOTHELIAL FUNCTION**

(30) Priority: 25.09.2023 JP 2023161748
(71) Applicant: Ezaki Glico Co., Ltd., Osaka-shi Osaka 555-8502 (JP)
(72) Inventor: AZUMA, Naoki, Osaka-shi, Osaka 555-8502 (JP); SAITO, Yasuo, Osaka-shi, Osaka 555-8502 (JP); NISHIJIMA, Tomohiko, Osaka-shi, Osaka 555-8502 (JP); NISHIHIRA, Jun, Ebetsu-shi, Hokkaido 069-8585 (JP); HONMA, Naoyuki, Ebetsu-shi, Hokkaido 069-8585 (JP); HASEDA, Akane, Ebetsu-shi, Hokkaido 069-8585 (JP); KATSUYAMA, Hiroyo, Ebetsu-shi, Hokkaido 069-8585 (JP)
(74) Representative: Hoffmann Eitle
(86) International application number: PCT/JP2024/034000
(87) International publication number: WO 2025/070420

(57) **Abstract**

In one embodiment, a problem to be solved by the present invention is to provide a novel means for improving vascular endothelial function. In one embodiment, the present invention provides a composition for improving vascular endothelial function, comprising bifidobacteria and water-soluble dietary fiber.

## Description

### Technical Field

The present invention relates to a composition for improving vascular endothelial function.

### Background Art

Cardiovascular disease (CVD) has become a leading cause of death worldwide, with a particularly large impact on low- and middle-income countries. An estimated 17.9 million people died of CVD in 2019, representing 32% of all deaths worldwide. Despite current prevention and treatment strategies, mortality from CVD is expected to increase further over the next decade. Arteriosclerosis is an inflammatory disease of the cardiovascular system characterized by a narrowing of the arterial lumen due to plaque formation, the progression of which directly leads to the development of CVD. Vascular endothelial dysfunction leads to plaque formation and disease progression, resulting in the development of atherosclerosis.

Similarly, CVD has been widely reported to be associated with metabolic syndrome. Metabolic syndrome is a clinical condition associated with at least three of the following metabolic risk factors: excess visceral fat (abdominal obesity), insulin resistance, hyperglycemia, hypertension, and dyslipidemia (i.e., high triglyceride levels and low high-density lipoprotein (HDL) cholesterol). These metabolic syndrome components have been found to impair endothelial function individually, and patients with metabolic syndrome are known to develop vascular endothelial dysfunction at a high frequency. Under these circumstances, there is a strong demand for the development of a novel means for improving vascular endothelial function.

### Citation List

### Patent Literature

PTL 1: JP2021-169517A
PTL 2: JP5881801B

### Non-patent Literature

NPL 1: J Biosci Bioeng. 2012; 113: 587-91.
NPL 2: Sci Rep. 2017; 7: 43522.
NPL 3: Food Sci Nutr. 2019; 7: 1828-37.
NPL 4: Scientific Reports.; volume 4, Article number: 4548 (2014).
NPL 5: Hypertension. 2018; 72: 1060-71.
NPL 6: Biosci Microbiota Food Health. 2016; 35(4): 163-171.
NPL 7: Genome Biol. 2022 Apr 14; 23(1): 95.
NPL 8: Microbiome. 2021 Apr 29; 9(1):95. doi: 10.1186/s40168-021-01048-3.
NPL 9: The Japanese Circulation Society and the Japanese Society for Vascular Failure (eds.), "Kekkan Fuzen no Seirigakuteki Shindan Shishin" [Physiological Diagnostic Guidelines for Vascular Failure], Life Science Publishing Co., Ltd., 2021.
NPL 10: Am J Clin Nutr. 2018; 107: 965-83.

### Summary of Invention

### Technical Problem

A problem to be solved by the present invention is to provide a novel means for improving vascular endothelial function.

### Solution to Problem

Under the circumstances described above, the present inventors conducted extensive research and found that a combination of bifidobacteria and water-soluble dietary fiber can improve vascular endothelial function. The present invention is based on such novel findings. Therefore, the present invention provides the following items:
Item 1. A composition for improving vascular endothelial function, comprising bifidobacteria and water-soluble dietary fiber.
Item 2. The composition according to Item 1, wherein the improvement of vascular endothelial function includes a decrease in blood LDL-C level or a decrease in PAI-1 level, or both.
Item 3. A composition for maintaining or preventing a decline in vascular flexibility or elasticity, comprising bifidobacteria and water-soluble dietary fiber.
Item 4. A composition for preventing arteriosclerosis, comprising bifidobacteria and water-soluble dietary fiber.
Item 5. A composition for improving kidney function, comprising bifidobacteria and water-soluble dietary fiber.
Item 6. The composition according to any one of Items 1 to 5, wherein the water-soluble dietary fiber comprises inulin.
Item 7. The composition according to any one of Items 1 to 6, wherein the bifidobacteria have an ability to proliferate in the gut.
Item 8. The composition according to any one of Items 1 to 7, wherein the bifidobacteria have an anti-metabolic syndrome effect.
Item 9. The composition according to any one of Items 1 to 8, which is a food product.
Item 10. The composition according to any one of Items 1 to 9, further comprising arginine.
Item 11. Use of bifidobacteria and water-soluble dietary fiber for the manufacture of a composition for improving vascular endothelial function.
Item 12. The use according to Item 11, wherein the improvement of vascular endothelial function includes a decrease in blood LDL-C level or a decrease in PAI-1 level, or both.
Item 13. Use of bifidobacteria and water-soluble dietary fiber for the manufacture of a composition for maintaining or preventing a decline in vascular flexibility or elasticity.
Item 14. Use of bifidobacteria and water-soluble dietary fiber for the manufacture of a composition for preventing arteriosclerosis.
Item 15. Use of bifidobacteria and water-soluble dietary fiber for the manufacture of a composition for improving kidney function.
Item 16. Use of water-soluble dietary fiber for the manufacture of a composition for improving vascular endothelial function, the composition comprising bifidobacteria.
Item 17. The use according to Item 16, wherein the improvement of vascular endothelial function includes a decrease in blood LDL-C level or a decrease in PAI-1 level, or both.
Item 18. Use of water-soluble dietary fiber for the manufacture of a composition for maintaining or preventing a decline in vascular flexibility or elasticity, the composition comprising bifidobacteria.
Item 19. Use of water-soluble dietary fiber for the manufacture of a composition for preventing arteriosclerosis, the composition comprising bifidobacteria.
Item 20. Use of water-soluble dietary fiber for the manufacture of a composition for improving kidney function, the composition comprising bifidobacteria.
Item 21. Use of bifidobacteria for the manufacture of a composition for improving vascular endothelial function, the composition comprising water-soluble dietary fiber.
Item 22. The use according to Item 21, wherein the improvement of vascular endothelial function includes a decrease in blood LDL-C level or a decrease in PAI-1 level, or both.
Item 23. Use of bifidobacteria for the manufacture of a composition for maintaining or preventing a decline in vascular flexibility or elasticity, the composition comprising water-soluble dietary fiber.
Item 24. Use of bifidobacteria for the manufacture of a composition for preventing arteriosclerosis, the composition comprising water-soluble dietary fiber.
Item 25. Use of bifidobacteria for the manufacture of a composition for improving kidney function, the composition comprising water-soluble dietary fiber.
Item 26. The use according to any one of Items 11 to 25, wherein the water-soluble dietary fiber comprises inulin.
Item 27. The use according to any one of Items 11 to 26, wherein the bifidobacteria have an ability to proliferate in the gut.
Item 28. The use according to any one of Items 11 to 27, wherein the bifidobacteria have an anti-metabolic syndrome effect.
Item 29. The use according to any one of Items 11 to 28, wherein the composition is a food product.
Item 30. The use according to any one of Items 11 to 29, wherein the composition further comprises arginine.
Item 31. A method for improving vascular endothelial function, comprising administering an effective amount of bifidobacteria and water-soluble dietary fiber to a subject in need thereof.
Item 32. The method according to Item 31, wherein the improvement of vascular endothelial function includes a decrease in blood LDL-C level or a decrease in PAI-1 level, or both.
Item 33. A method for maintaining or preventing a decline in vascular flexibility or elasticity, comprising administering an effective amount of bifidobacteria and water-soluble dietary fiber to a subject in need thereof.
Item 34. A method for preventing arteriosclerosis, comprising administering an effective amount of bifidobacteria and water-soluble dietary fiber to a subject in need thereof.
Item 35. A method for improving kidney function, comprising administering an effective amount of bifidobacteria and water-soluble dietary fiber to a subject in need thereof.
Item 36. The method according to any one of Items 31 to 35, wherein the water-soluble dietary fiber comprises inulin.
Item 37. The method according to any one of Items 31 to 36, wherein the bifidobacteria have an ability to proliferate in the gut.
Item 38. The method according to any one of Items 31 to 37, wherein the bifidobacteria have an anti-metabolic syndrome effect.
Item 39. The method according to any one of Items 31 to 38, wherein the bifidobacteria and the water-soluble dietary fiber are ingested in the form of a food product.
Item 40. The method according to any one of Items 31 to 39, further comprising administering arginine.
Item 41. A combination of bifidobacteria and water-soluble dietary fiber for use in improving vascular endothelial function.
Item 42. The combination according to Item 41, wherein the improvement of vascular endothelial function includes a decrease in blood LDL-C level or a decrease in PAI-1 level, or both.
Item 43. A combination of bifidobacteria and water-soluble dietary fiber for use in maintaining or preventing a decline in vascular flexibility or elasticity.
Item 44. A combination of bifidobacteria and water-soluble dietary fiber for use in preventing arteriosclerosis.
Item 45. A combination of bifidobacteria and water-soluble dietary fiber for use in improving kidney function.
Item 46. The combination according to any one of Items 41 to 45, wherein the water-soluble dietary fiber comprises inulin.
Item 47. The combination according to any one of Items 41 to 46, wherein the bifidobacteria have an ability to proliferate in the gut.
Item 48. The combination according to any one of Items 41 to 47, wherein the bifidobacteria have an anti-metabolic syndrome effect.
Item 49. The combination according to any one of Items 41 to 48, which is a food product.
Item 50. The combination according to any one of Items 41 to 49, further comprising arginine.
Item 51. Water-soluble dietary fiber for use in combination with bifidobacteria in improving vascular endothelial function.
Item 52. The water-soluble dietary fiber according to Item 51, wherein the improvement of vascular endothelial function includes a decrease in blood LDL-C level or a decrease in PAI-1 level, or both.
Item 53. Water-soluble dietary fiber for use in combination with bifidobacteria in maintaining or preventing a decline in vascular flexibility or elasticity.
Item 54. Water-soluble dietary fiber for use in combination with bifidobacteria in preventing arteriosclerosis.
Item 55. Water-soluble dietary fiber for use in combination with bifidobacteria in improving kidney function.
Item 56. The water-soluble dietary fiber according to any one of Items 51 to 55, wherein the water-soluble dietary fiber comprises inulin.
Item 57. The water-soluble dietary fiber according to any one of Items 51 to 56, wherein the bifidobacteria have an ability to proliferate in the gut.
Item 58. The water-soluble dietary fiber according to any one of Items 51 to 57, wherein the bifidobacteria have an anti-metabolic syndrome effect.
Item 59. The water-soluble dietary fiber according to any one of Items 51 to 58, which is a food product.
Item 60. The water-soluble dietary fiber according to any one of Items 51 to 59, for use in further combination with arginine.
Item 61. Bifidobacteria for use in combination with water-soluble dietary fiber in improving vascular endothelial function.
Item 62. The bifidobacteria according to Item 61, wherein the improvement of vascular endothelial function includes a decrease in blood LDL-C level or a decrease in PAI-1 level, or both.
Item 63. Bifidobacteria for use in combination with water-soluble dietary fiber in maintaining or preventing a decline in vascular flexibility or elasticity.
Item 64. Bifidobacteria for use in combination with water-soluble dietary fiber in preventing arteriosclerosis.
Item 65. Bifidobacteria for use in combination with water-soluble dietary fiber in improving kidney function.
Item 66. The bifidobacteria according to any one of Items 61 to 65, wherein the water-soluble dietary fiber comprises inulin.
Item 67. The bifidobacteria according to any one of Items 61 to 66, which have an ability to proliferate in the gut.
Item 68. The bifidobacteria according to any one of Items 61 to 67, which have an anti-metabolic syndrome effect.
Item 69. The bifidobacteria according to any one of Items 61 to 68, which is a food product.
Item 70. The bifidobacteria according to any one of Items 61 to 69, for use in further combination with arginine.

### Advantageous Effects of Invention

The present invention can provide a novel means for improving vascular endothelial function.

### Brief Description of Drawings

Fig. 1 shows a flowchart of the process from subject enrollment to analysis in the Examples.
Fig. 2 shows the effect of intake of a test food containing GCL2505 and inulin on the fecal microbiota in a subgroup-analysis population (test food group: n = 22, placebo group: n = 21). The box plots represent the 5th percentile, 95th percentile, interquartile range (25% to 75%), and median. A) Alpha-diversity (Chao1). B) Beta-diversity (principal components analysis of genus-level Bray-Curtis distance). C) Relative abundance of *B. animalis* at week 12. Data were analyzed by LinDA.

### Description of Embodiments

### Composition for Improving Vascular Endothelial Function

The present invention provides a composition for improving vascular endothelial function, comprising bifidobacteria and water-soluble dietary fiber. In the present invention, examples of the types of bifidobacteria include, but are not particularly limited to, *Bifidobacterium animalis*, *Bifidobacterium adolescentis*, *Bifidobacterium bifidum*, *Bifidobacterium breve*, *Bifidobacterium catenulatum*, *Bifidobacterium longum*, and *Bifidobacterium pseudocatenulatum*, with *Bifidobacterium animalis* being preferred. Among *Bifidobacterium animalis*, *Bifidobacterium animalis* subsp. *lactis* is preferred. The bifidobacteria are preferably those that have an ability to proliferate in the gut. More specifically, the bifidobacteria are preferably those capable of increasing the total number of bifidobacteria in the gut. Whether or not they have the ability to proliferate in the gut can be determined, for example, by measuring the number of bifidobacteria in a fecal sample using real-time PCR. More specifically, the measurement and evaluation can be performed according to the method described in NPL 1. In the present invention, if the proliferation in the gut is confirmed by the above method, the bifidobacteria can be selected as preferable bifidobacteria of the present invention. It is preferable that the bacteria can proliferate so that the number of bifidobacteria becomes two-fold or more, preferably five-fold or more, and more preferably ten-fold or more compared with the number of bacteria ingested into the body when measured by the above method. Furthermore, in the present invention, the bifidobacteria are preferably those that have an anti-metabolic syndrome effect. The anti-metabolic syndrome effect can be confirmed, for example, by measuring visceral fat area. More specifically, the measurement and evaluation can be performed according to the method described in NPL 6. In the present invention, if a reduction in visceral fat area is confirmed by the above method, the bifidobacteria can be selected as preferable bifidobacteria of the present invention. The bifidobacteria are preferably those that reduce visceral fat area to 0.98 times or less, and more preferably 0.96 times or less when measured by the above method. In a preferable embodiment of the present invention, examples of bifidobacteria include GCL2505 belonging to *Bifidobacterium animalis* subsp. *lactis*. The GCL2505 strain is deposited under the accession number FERM ABP-21918. The strain was deposited on February 17, 2010, with the Patent Microorganisms Depositary, National Institute of Technology and Evaluation (NITE) (Room No. 120, 2-5-8 Kazusakamatari, Kisarazushi, Chiba Prefecture, Japan, 292-0818) under the Budapest Treaty.

The number of bifidobacteria in the composition of the present invention is not limited and can be appropriately set within the range of, for example, 10 million/g, preferably 30 million/g, more preferably 50 million/g, even more preferably 80 million/g or more, and particularly preferably 100 million/g or more. The upper limit is not particularly limited, and is, for example, 10 trillion/g or less, 100 billion/g or less, or 10 billion/g or less. In a typical embodiment of the present invention, live bifidobacteria are used.

Examples of the water-soluble dietary fiber include inulin, indigestible dextrin, water-soluble soybean polysaccharides, polydextrose, sodium alginate, psyllium, fucoidan, laminaran, sodium carboxymethyl cellulose, pullulan, curdlan, and low-molecular-weight hemicellulose, with inulin being preferred. These water-soluble dietary fibers may be used singly or in a combination of multiple types.

The content of the water-soluble dietary fiber in the composition of the present invention is not limited and can be set within the range of, for example, 0.5 to 10 wt%, preferably 1 to 5 wt%, and more preferably 1.5 to 3 wt%, based on the total mass of the composition.

In the present invention, a combination of bifidobacteria and water-soluble dietary fiber, which are active ingredients of the present invention, may be used in the form of a composition for improving vascular endothelial function. Alternatively, the combination may be used in the form of a composition in combination with various carriers that are pharmaceutically acceptable or that can be added to food (e.g., isotonic agents, chelating agents, stabilizers, pH adjusters, preservatives, antioxidants, solubilizing agents, thickeners, excipients, and binders). In an embodiment in which various carriers are contained, the total content of the bifidobacteria and the water-soluble dietary fiber in the composition is not limited, and can be appropriately set within the range of, for example, 50 mass% or more, 60 mass% or more, 70 mass% or more, 80 mass% or more, 90 mass% or more, 95 mass% or more, 99 mass% or more, and the like.

Examples of isotonic agents include sugars, such as glucose, trehalose, lactose, fructose, mannitol, xylitol, and sorbitol; polyhydric alcohols, such as glycerin, polyethylene glycol, and propylene glycol; and inorganic salts, such as sodium chloride, potassium chloride, and calcium chloride. These isotonic agents can be used singly or in a combination of two or more.

Examples of chelating agents include edetates, such as disodium edetate, calcium disodium edetate, trisodium edetate, tetrasodium edetate, and calcium edetate, as well as ethylenediaminetetraacetate, nitrilotriacetic acid or its salt, sodium hexametaphosphate, and citric acid. These chelating agents can be used singly or in a combination of two or more.

Examples of stabilizers include sodium bisulfite.

Examples of pH adjusters include acids, such as hydrochloric acid, carbonic acid, acetic acid, and citric acid, alkali metal hydroxides, such as sodium hydroxide and potassium hydroxide, alkali metal carbonates or hydrogencarbonates, such as sodium carbonate, alkali metal acetates, such as sodium acetate, alkali metal citrates, such as sodium citrate, and bases, such as trometamol. These pH adjusters can be used singly or in a combination of two or more.

Examples of preservatives include sorbic acid, potassium sorbate, p-hydroxybenzoates, such as methyl p-hydroxybenzoate, ethyl p-hydroxybenzoate, propyl p-hydroxybenzoate, and butyl p-hydroxybenzoate, quaternary ammonium salts, such as chlorhexidine gluconate, benzalkonium chloride, benzethonium chloride, and cetylpyridinium chloride, alkyl polyaminoethyl glycine, chlorobutanol, polyquaternium, polyhexamethylene biguanide, and chlorhexidine. These preservatives can be used singly or in a combination of two or more.

Examples of antioxidants include sodium bisulfite, dried sodium sulfite, sodium pyrosulfite, and mixed tocopherol concentrate. These antioxidants can be used singly or in a combination of two or more.

Examples of solubilizing agents include sodium benzoate, glycerin, D-sorbitol, glucose, propylene glycol, hydroxypropyl methylcellulose, polyvinylpyrrolidone, macrogol, and D-mannitol. These solubilizing agents can be used singly or in a combination of two or more.

Examples of thickeners include polyethylene glycol, methyl cellulose, ethyl cellulose, carmellose sodium, xanthan gum, sodium chondroitin sulfate, hydroxyethyl cellulose, hydroxypropyl cellulose, hydroxypropyl methylcellulose, polyvinylpyrrolidone, and polyvinyl alcohol. These thickeners can be used singly or in a combination of two or more.

Examples of excipients include lactose, corn starch, L-cysteine, trehalose, maltitol, and sorbitol. These excipients can be used singly or in a combination of two or more.

Examples of binders include crystalline cellulose, starch, sucrose, hydroxypropyl cellulose, gelatin, powdered gum arabic, polyvinylpyrrolidone, pullulan, dextrin, cyclodextrin, methyl cellulose, ethyl cellulose, hydroxymethyl cellulose, hydroxypropyl cellulose, hydroxypropyl methyl cellulose, polyvinyl alcohol, and polyethylene glycol. These binders can be used singly or in a combination of two or more.

Intake of the composition of the present invention by a subject (preferably a mammal, such as a human) can improve vascular endothelial function. PTL 1 discloses in Example 2 that intake of a set of live bacteria powder of *Bifidobacterium animalis* subsp. *lactis* LKM512 strain (approximately 6 x 10⁹ cfu/package × 1 package) combined with arginine tablets (100 mg/tablet × 3 tablets) twice a day, after breakfast and dinner, for 8 weeks resulted in improved vascular endothelial function (EndoPAT measurement values). However, PTL 1 also discloses that vascular endothelial function did not improve in the placebo group in which the LKM512 strain was combined with starch instead of the arginine tablets. Therefore, PTL 1 discloses that vascular endothelial function did not improve when bifidobacteria were used alone. Furthermore, there have been no reports so far, including in PTL 1, that vascular endothelial function can be improved by combining bifidobacteria with water-soluble dietary fiber. Accordingly, the effects of the present invention are unpredictable from the prior art.

PTL 2 discloses that arginine intake increases the concentration of polyamines (putrescine) in intestinal bacterial metabolites, and NPL 4 discloses that intake of the LKM512 strain in combination with arginine results in upregulation of polyamines (putrescine). Therefore, it is suggested that the increase in EndoPAT measurement values as a result of the intake of the LKM512 strain in combination with arginine is due to the production of polyamines (putrescine) through arginine uptake. The effects of the present invention achieved by combining bifidobacteria with water-soluble dietary fiber is not due to the production of polyamines (putrescine), and vascular endothelial function can be improved by a mechanism completely different from arginine administration; therefore, the combination of the present invention is useful.

The intake amount of the composition of the present invention is not limited, and the daily intake of the bifidobacteria as the active ingredient can be appropriately set within the range of, for example, 10 million to 10 trillion, preferably 100 million to 1 trillion, more preferably 1 billion to 100 billion, even more preferably 5 billion to 30 billion, and particularly preferably 10 billion to 20 billion bifidobacteria. While the intake amount of the composition of the present invention based on the weight of bifidobacteria is also not limited, the daily intake of bifidobacteria as the active ingredient can be appropriately set within the range of, for example, 1 mg to 10 g, preferably 10 mg to 1000 mg, more preferably 40 mg to 300 mg, and even more preferably 200 mg or more.

In addition to the bifidobacteria described above, the composition of the present invention may further comprise a substance known to improve vascular endothelial function. Examples of substances known to improve vascular endothelial function include arginine, pine bark-derived procyanidin, black soybean polyphenol, and skipjack tuna-derived elastin peptide. These substances can be used singly or in a combination of two or more.

The composition of the present invention is preferably an oral composition. The oral composition includes food or drink compositions and pharmaceutical compositions. In the present invention, the food or drink compositions include health-functional foods (foods with nutrient function claims, foods for specified health uses, and foods with function claims).

Examples of food or drink compositions include beverages, such as vegetable juice beverages, fruit juice beverages, mixed vegetable and fruit juice beverages, fermented milk beverages, and almond-containing beverages; and foods, such as ice cream, sherbets, candies, gummies, almond-containing foods, biscuits (e.g., cream sandwich biscuits), chocolate (including quasi-chocolate), and fermented dairy products (yogurt and cheese). The ice cream is preferably lacto-ice cream (such as lacto-ice cream containing fermented milk). The quasi-chocolate includes corn-containing quasi-chocolate. The dairy products include fermented milk, ice cream, and milk beverages. The food or drink composition of the present invention also includes supplements and the like.

### Other Compositions and the like

Although the present invention is described above with reference to some embodiments, the present invention is not limited to these embodiments. For example, as demonstrated in the Examples described below, according to the present invention, a combination of bifidobacteria and water-soluble dietary fiber can improve vascular flexibility (typically, the degree of vascular dilatation after constriction). In the present invention, the term "vascular" in the phrases "vascular endothelial function," "vascular flexibility," and the like is not particularly limited and includes, for example, blood vessels of the brain, heart, and the like. Further, in the present invention, "vascular" and "blood vessels" are not limited and include, for example, arteries. A combination of bifidobacteria and water-soluble dietary fiber can improve vascular elasticity. Therefore, the combination can be expected to have a preventive effect on the conditions of such blood vessels affected by aging and the like. Therefore, in one embodiment, the present invention provides a composition for maintaining or preventing a decline in vascular flexibility or elasticity, comprising bifidobacteria and water-soluble dietary fiber. Vascular flexibility (or elasticity) can be measured, for example, by the FMD (flow-mediated dilation) test. Maintaining or preventing a decline in vascular flexibility (or elasticity) means that, for example, the FMD value is maintained at 4% or more (preferably 6% or more, and more preferably 7% or more) or increased from an FMD value of 4% or more (preferably 6% or more, and more preferably 7% or more) (NPL 5). As demonstrated in the Examples below, the combination of bifidobacteria and water-soluble dietary fiber improves vascular endothelial function and reduces the blood LDL-c level. Therefore, according to the present invention, the risk of arteriosclerosis can be reduced. Therefore, in one embodiment, the present invention provides a composition for preventing arteriosclerosis, comprising bifidobacteria and water-soluble dietary fiber. Furthermore, as demonstrated in the Examples below, the combination of bifidobacteria and water-soluble dietary fiber improves vascular flexibility as measured by the FMD test; since there have been reports on correlations between FMD measurement values and kidney function, improving vascular endothelial dysfunction can reduce the risk of developing kidney disease. Therefore, the present invention provides a composition for improving kidney function, comprising bifidobacteria and water-soluble dietary fiber. In these embodiments, the types and amounts of the bifidobacteria and water-soluble dietary fiber used, the conditions of other ingredients, and the like may be the same as those described in the "Composition for Improving Vascular Endothelial Function" section.

In another embodiment, the present invention provides use of bifidobacteria and water-soluble dietary fiber for the manufacture of a composition for improving vascular endothelial function; use of bifidobacteria and water-soluble dietary fiber for the manufacture of a composition for maintaining or preventing a decline in vascular flexibility or elasticity; use of bifidobacteria and water-soluble dietary fiber for the manufacture of a composition for preventing arteriosclerosis; use of bifidobacteria and water-soluble dietary fiber for the manufacture of a composition for improving kidney function; use of bifidobacteria for the manufacture of a composition for improving vascular endothelial function, the composition comprising water-soluble dietary fiber; the use according to item 21, wherein the improvement of vascular endothelial function includes a decrease in blood LDL-C level or a decrease in PAI-1 level, or both; use of bifidobacteria for the manufacture of a composition for maintaining or preventing a decline in vascular flexibility or elasticity, the composition comprising water-soluble dietary fiber; use of bifidobacteria for the manufacture of a composition for preventing arteriosclerosis, the composition comprising water-soluble dietary fiber; use of bifidobacteria for the manufacture of a composition for improving kidney function, the composition comprising water-soluble dietary fiber; a method for improving vascular endothelial function, comprising administering an effective amount of bifidobacteria and water-soluble dietary fiber to a subject in need thereof; a method for maintaining or preventing a decline in vascular flexibility or elasticity, comprising administering an effective amount of bifidobacteria and water-soluble dietary fiber to a subject in need thereof; a method for preventing arteriosclerosis, comprising administering an effective amount of bifidobacteria and water-soluble dietary fiber to a subject in need thereof; a method for improving kidney function, comprising administering an effective amount of bifidobacteria and water-soluble dietary fiber to a subject in need thereof; a combination of bifidobacteria and water-soluble dietary fiber for use in improving vascular endothelial function; a combination of bifidobacteria and water-soluble dietary fiber for use in maintaining or preventing a decline in vascular flexibility or elasticity; a combination of bifidobacteria and water-soluble dietary fiber for use in preventing arteriosclerosis; a combination of bifidobacteria and water-soluble dietary fiber for use in improving kidney function; water-soluble dietary fiber for use in combination with bifidobacteria in improving vascular endothelial function; water-soluble dietary fiber for use in combination with bifidobacteria in maintaining or preventing a decline in vascular flexibility or elasticity; water-soluble dietary fiber for use in combination with bifidobacteria in preventing arteriosclerosis; water-soluble dietary fiber for use in combination with bifidobacteria in improving kidney function; bifidobacteria for use in combination with water-soluble dietary fiber in improving vascular endothelial function; bifidobacteria for use in combination with water-soluble dietary fiber in maintaining or preventing a decline in vascular flexibility or elasticity; bifidobacteria for use in combination with water-soluble dietary fiber in preventing arteriosclerosis; and bifidobacteria for use in combination with water-soluble dietary fiber in improving kidney function.

In these embodiments, the details (type, amount, etc.) regarding the bifidobacteria, the water-soluble dietary fiber, and optional other ingredients, the method of use (pharmaceutical products, food and beverages, etc.), and the purpose of use (improvement of vascular endothelial function, prevention of arteriosclerosis, improvement of kidney function, etc.) are the same as those described above. In the present invention, examples of subjects suitable for administration (or ingestion) of the bifidobacteria and water-soluble dietary fiber include mammals, such as humans, mice, rats, and guinea pigs (preferably humans). In the case of human subjects, examples include, but are not particularly limited to, humans who do not have arteriosclerosis, kidney disease, hypertension, or the like. While the age of the subjects is also not particularly limited, examples of the age include age groups in which vascular endothelial function, kidney function, and the like are generally considered to decline with aging. In one non-limiting preferred embodiment, the subjects of the present invention include humans (particularly middle-aged and elderly humans) who have not yet developed arteriosclerosis, kidney disease, hypertension, or the like.

Below, specific embodiments of the present invention will be described in more detail with reference to Examples. However, the present invention is not limited to these Examples.

### Examples

A randomized, double-blind, placebo-controlled, parallel-group comparative study was conducted to examine the effects of GCL2505 and inulin, which have an anti-metabolic syndrome effect, on vascular endothelial function, in order to evaluate the potential of reducing the risk of arteriosclerosis.

### Subjects (Analysis Target Population)

In this study, written informed consent was obtained from all participants based on the Declaration of Helsinki. The participants were Japanese men and women aged 40 to under 65 showing a trend toward reduced vascular endothelial function, who satisfied the inclusion criteria, did not satisfy the exclusion criteria, and were deemed eligible to participate by the principal investigator. The inclusion criteria were as follows:
(1) individuals who fully understand the significance, content, and purpose of the test and provide written informed consent to participate in the test;
(2) Japanese men and women aged 40 to under 65 at the time of screening;
(3) individuals with high pentraxin 3 (PTX3) levels at the time of screening; and
(4) individuals with a high LDL-cholesterol-to-HDL-cholesterol ratio (L/H ratio) at the time of screening.
The exclusion criteria were as follows:
(1) individuals who have difficulty with FMD measurement on the right arm;
(2) individuals receiving treatment, medication, or lifestyle guidance from a medical doctor for hypertension, dyslipidemia, or diabetes;
(3) individuals with a history of bronchial disease, such as asthma, tuberculosis, or pleurisy;
(4) individuals using a pacemaker or defibrillator;
(5) individuals suffering from serious cerebrovascular disease, heart disease, liver disease, renal disease, gastrointestinal disease, or infectious disease requiring notification;
(6) individuals with a history of major gastrointestinal surgery, such as gastrectomy, gastrointestinal suture, or intestinal resection;
(7) individuals with significant abnormalities in blood pressure measurement, physical measurements, or blood tests;
(8) individuals with severe anemia;
(9) pre- or postmenopausal women with significant physical changes;
(10) individuals at risk of exhibiting allergic symptoms to drugs or foods (particularly milk ingredients);
(11) individuals taking or planning to take antibiotics from 12 weeks before the start of intake to the end of the test;
(12) individuals who regularly intake medications that affect bowel movements (e.g., probiotics, laxatives, antidiarrheals);
(13) individuals who are unable to stop consuming yogurt, lactic acid bacteria beverages, or health foods with intestinal regulation effects (containing ingredients, such as lactic acid bacteria, bifidobacteria, oligosaccharides, and dietary fiber-enriched foods) during the test period;
(14) smokers, heavy drinkers, or individuals with extremely irregular lifestyles;
(15) women who donated 400 mL of blood within 16 weeks before the start of intake, men who donated 400 mL of blood within 12 weeks before the start of intake, individuals who donated 200 mL of blood within 4 weeks before the start of intake, or individuals who donated blood components within 2 weeks before the start of intake;
(16) women who are pregnant or suspected to be pregnant, or who are breastfeeding;
(17) individuals participating in other clinical tests, observational studies, clinical trials, or home-use tests from 4 weeks before the start of the test period to the end of intake; and
(18) any other individuals deemed ineligible by the principal investigator.

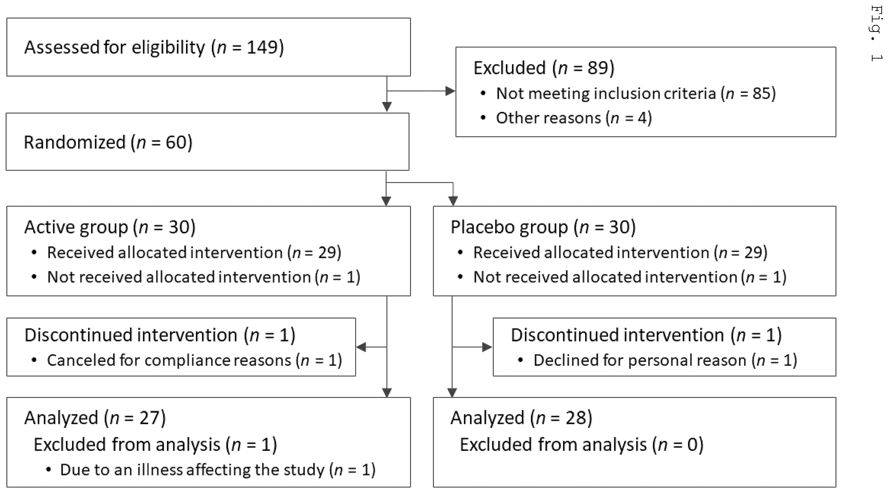

An overview of the test period is shown in the flowchart in Fig. 1. In this study, 149 participants were screened. As a result of the screening, 60 participants were eligible: 30 were assigned to a test food group and 30 were assigned to a placebo group. By the end of the test, three participants had withdrawn for personal reasons (n = 1 from the test food group, n = 2 from the placebo group). In addition, one participant was determined to be discontinued from a compliance perspective. After the completion of the entire test, one participant was dropped due to a confirmed illness unrelated to the test that might have affected the results. Therefore, a total of 55 subjects (27 in the test food group and 28 in the placebo group) were included in the analysis target population.

The participants in the test food group (Active) and the placebo group (Placebo) each consumed a drink with the composition shown in Table 1 below once daily. Although dietary menus other than the drinks were not restricted, prohibited items were set, including prohibiting the intake of foods containing lactic acid bacteria. The intake of the drink was continued for 84 days.

**Table 1. Nutritional details of the test drinks**

| | Active | Placebo |
|---|---|---|
| Energy (kcal) | 52.0 | 48.0 |
| Moisture (g) | 84.9 | 86.9 |
| Protein (g) | 2.8 | 2.8 |
| Fat (g) | 0.1 | 0.1 |
| Carbohydrate (g) | 11.2 | 9.1 |
| Ash (g) | 1.1 | 1.1 |

The active drink contained 2 g inulin and 1.0 × 10¹⁰ colony-forming units GCL2505

Table 2 below shows an overview of the background and safety evaluation of the subjects in the full analysis target population. Table 3 shows the measurement results of FMD and blood components in the test food group (Active) and the placebo group (Placebo) in the full analysis target population.

**Table 2. Summary of the subjects' characteristics and safety in the efficacy analysis population.**

| | Active | Placebo | *P*-value |
|---|---|---|---|
| Age, years | 50.37 (7.47) | 50.04 (6.33) | 0.859 |
| Number of women | 24 of total 27 | 24 of total 28 | - |
| Systolic blood pressure, mmHg | 112.81 (12.52) | 114.86 (13.40) | 0.562 |
| Diastolic blood pressure, mmHg | 77.63 (9.23) | 79.21 (8.68) | 0.515 |
| Heart rate, bpm | 69.22 (11.24) | 65.36 (6.75) | 0.131 |
| Height, cm | 159.66 (5.92) | 160.56 (8.03) | 0.637 |
| Body weight, kg | 54.27 (6.78) | 54.24 (9.84) | 0.987 |
| Body fat, % | 29.11 (6.51) | 27.41 (6.37) | 0.332 |
| Body mass index, kg/m² | 21.29 (2.56) | 20.93 (2.59) | 0.609 |

All data are presented as mean (SD), except for the number of women.

*P*-value shows the inter-group difference (active group vs. placebo group; Student's *t*-test).

**Table 3. Effects of the test drinks on FMD and blood parameters in the efficacy analysis population.**

| | | Week 8 | | Week 12 | |
|---|---|---|---|---|---|
| | | Change | *P*-value | Change | *P*-value |
| FMD, % | Active | 0.61 (2.13) | | 1.05 (2.21) | |
| | Placebo | 0.53 (2.24) | 0.899 | -0.26 (2.87) | 0.065 |
| Total cholesterol, mg/dL | Active | -0.74 (13.05) | | -2.78 (20.64) | |
| | Placebo | 2.32 (13.65) | 0.399 | 1.48 (14.19) | 0.382 |
| LDL-c, mg/dL | Active | -2.89 (9.97) | | -3.44 (14.51) | |
| | Placebo | 2.82 (10.36) | 0.042 | 1.41 (10.01) | 0.159 |
| HDL-c, mg / dL | Active | 2.74 (6.71) | | 2.11 (8.25) | |
| | Placebo | 1.07 (5.89) | 0.332 | 1.33 (7.47) | 0.718 |
| Triglycerides, mg/dL | Active | -6.11 (27.74) | | -4.59 (23.99) | |
| | Placebo | -4.93 (22.38) | 0.863 | -0.96 (18.11) | 0.533 |
| LDL-c/HDL-c | Active | -0.09 (0.21) | | -0.10 (0.24) | |
| | Placebo | 0.01 (0.13) | 0.035 | -0.01 (0.14) | 0.120 |
| PAI-1, ng/mL | Active | -2.07 (5.34) | | -1.22 (4.41) | |
| | Placebo | 0.39 (2.41) | 0.035 | 0.89 (2.97) | 0.045 |
| TMAO, µg/mL | Active | 0.10 (0.39) | | 0.29 (0.82) | |
| | Placebo | -0.03 (0.34) | 0.211 | 0.18 (0.78) | 0.608 |

All data are presented as mean (SD).

The data for weeks 8 and 12 show the amount of change from week 0, respectively.

*P*-value shows the inter-group difference (change value of active group vs. placebo group; Student's *t*-test).

Table 4 below shows the measurement results of FMD and blood components in the test food group (Active) and the placebo group (Placebo) in the population of subjects with an FMD (%) of 4% or more at week 0.

**Table 4**

| Table 4. Effects of the test drinks on FMD and blood parameters in a population of subgroup analysis (active group: *n* = 24; placebo group: *n =* 23). | | | | | |
|---|---|---|---|---|---|
| | | Week 8 | | Week 12 | |
| | | Change | *P*-value | Change | *P*-value |
| FMD, % | Active | 0.35 (1.98) | | 0.88 (2.23) | |
| | Placebo | 0.12 (1.95) | 0.692 | -0.68 (2.81) | 0.046 |
| Total cholesterol, mg/dL | Active | -2.75 (11.51) | | -3.75 (21.09) | |
| | Placebo | 3.87 (13.74) | 0.081 | 3.00 (13.55) | 0.200 |
| LDL-c, mg/dL | Active | -4.17 (9.18) | | -4.29 (15.14) | |
| | Placebo | 3.57 (10.93) | 0.012 | 2.14 (9.73) | 0.092 |
| HDL-c, mg/dL | Active | 2.08 (6.50) | | 2.04 (7.39) | |
| | Placebo | 1.74 (5.79) | 0.849 | 2.00 (7.98) | 0.985 |
| Triglycerides, mg/dL | Active | -6.08 (29.03) | | -4.42 (25.02) | |
| | Placebo | -6.52 (23.71) | 0.955 | -2.41 (18.43) | 0.757 |
| LDL-c/HDL-c | Active | -0.10 (0.21) | | -0.10 (0.24) | |
| | Placebo | 0.01 (0.14) | 0.041 | -0.02 (0.14) | 0.150 |
| PAI-1, ng/mL | Active | -1.83 (5.25) | | -1.25 (4.66) | |
| | Placebo | 0.52 (2.61) | 0.058 | 0.86 (3.12) | 0.076 |
| TMAO, µg/mL | Active | 0.12 (0.41) | | 0.34 (0.86) | |
| | Placebo | -0.02 (0.37) | 0.228 | 0.06 (0.59) | 0.197 |

All data are presented as mean (SD).

The data for weeks 8 and 12 show the amount of change from week 0, respectively.

*P*-value shows the inter-group difference (change value of active group vs. placebo group; Student's *t*-test).

### Safety Assessment

The safety of the analysis target population (Table 2) was confirmed, and there were no clinically significant problems. In addition, no side effects or medically problematic adverse events were observed. Therefore, the dairy drinks used in the test were considered safe for consumption.

### FMD

FMD was measured on Day 0, Day 56, and Day 84 of the test according to the following method. For the measurement, an UNEX-EF18VG (UNEX Corporation) was used. The right arm was used for the measurement. First, the vessel diameter of the right brachial artery was measured at rest, and the right forearm was then occluded for 5 minutes. After releasing the occlusion, the vessel diameter at maximal dilation was measured again. The percentage dilation of the vessel diameter relative to resting diameter was expressed as FMD (%).

An increase in FMD (%) in the test food group at week 12 was confirmed in the population of subjects with an FMD (%) of 4% or more at week 0 (0.88% ± 2.23%). Furthermore, there was a statistically significant difference in the change in FMD (%) from week 0 to week 12 between the test food group and the placebo group (-0.68% ± 2.81%) (*p* = 0.046 by Student's *t*-test) (Table 4). In the analysis target population, an increase in FMD (%) was observed in the test food group at week 12 (1.05 ± 2.21%). The change in FMD (%) from week 0 to week 12 in the test food group was greater than that in the placebo group (-0.26% ± 2.87%) (*p* = 0.065 by Student's *t*-test) (Table 3).

### Fecal Microbiota

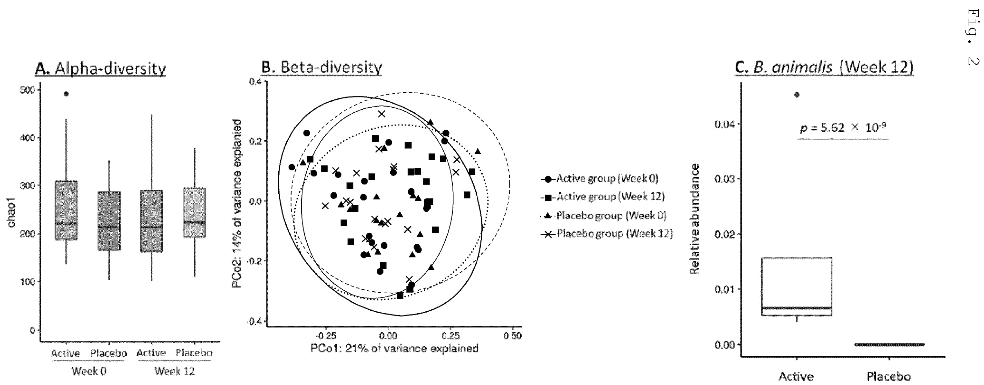

The fecal microbiota was examined by shotgun metagenomic analysis in the subgroup-analysis population at weeks 0 and 12. Alpha-diversity (Chao1) and beta-diversity analyses were performed to compare the fecal microbiota of both groups. Interestingly, no differences were observed between or within both groups at weeks 0 and 12 (Figs. 2A and B). However, the relative abundance of *B. animalis* in the intestine of the test food group was 0.061% ± 0.002% at week 0 and increased to 1.503% ± 0.018% at week 12. In the placebo group, its abundance was 0.085% ± 0.004% at week 0 and 0.014% ± 0.001% at week 12. Differences in abundance between the two groups were assessed using the Linear Model for Differential Abundance (LinDA) method for compositional data, with *p*-values corrected using the Benjamini-Hochberg procedure to minimize the false discovery rate (NPL 7). At week 12, there was a statistically significant difference in the relative abundance of *B. animalis* between the test food group and the placebo group (*p* = 5.62 × 10⁻⁹) (Fig. 2C).

### Test Methods

### Fecal Sample Collection

Fecal samples were collected by the participants at home at weeks 0, 8, and 12 from 3 days before to the morning of the scheduled visit date, using a fecal container containing RNAlater Stabilization Solution (Invitrogen, Carlsbad, CA). The fecal samples were stored in a refrigerator at home and brought to the laboratory on the day of testing while being cooled with a refrigerant.

### Fecal DNA Extraction

The bacterial DNA was extracted from 10-fold dilutions of the fecal samples using an ISOSPIN Fecal DNA Kit (Nippon Gene Co., Ltd., Tokyo, Japan) according to the method described above (NPL 8). Specifically, the sample (here, 200 µL of fecal dilution), 700 µL of FE1 buffer, and 10 µL of RNase were added to the provided tubes containing beads. FastPrep-24 (MP Biomedicals, Irvine, CA) was used for bead-beating for 1 minute at a rate of 6 m/s to crush the cells. Bead-beating was performed 3 times, during which the samples were kept at room temperature for 5 minutes. Subsequently, 90 µL of FE2 buffer was added, and the samples were centrifuged at 12,000× g for 15 minutes. The supernatant (up to 500 µL) was collected and mixed with FB buffer and isopropanol, each at 0.4× the volume of the supernatant obtained. Finally, the samples were loaded onto spin columns and washed according to the manufacturer's instructions. The purified DNA was eluted with 50 µL of Tris-EDTA buffer (pH 8.0).

### Shotgun Library Construction and Sequencing

Unless otherwise stated, metagenomic sequencing libraries were constructed using a QIAseq FX DNA Library Kit (Qiagen, Hilden, Germany) according to the manufacturer's instructions. Briefly, each enzymatic fragmentation reaction (50 µL) included 10× FX buffer, 10 µL of FX enzyme mix, and 500 ng of DNA template, and the reaction was incubated at 32°C for 9 minutes. For adapter ligation, 5 µL of adapter was added, along with 20 µL of DNA ligase buffer, 10 µL of DNA ligase, and 15 µL of RNase-free H₂O, and the reaction was incubated at 20°C for 15 minutes. Adapter-ligated fragments were purified and size-selected with an Agencourt AMPure XP PCR purification system (Beckman Coulter, Brea, CA) sequentially using 1 and 0.8 volumes of bead solution, and eluted with 10 mM Tris-HCl buffer.

### Quality Control of Metagenomic Reads

Quality control of the metagenomic reads and adapter sequence trimming were performed using fastp (version 0.20.0). Reads less than 50 bp were excluded from further analysis. The remaining reads were mapped to the human (hg38) and phiX bacteriophage genomes using minimap2 (version 2.17), and the mapped reads were excluded. Reads whose pairs were excluded in the filtering steps were also removed.

### Construction of Non-redundant Gene Set and Functional Annotation

A non-redundant gene set was constructed based on HMP and population-level metagenomics data. All genes were clustered with a 95% identity threshold using cd-hit (version 4.8.1). Functional annotation of the non-redundant genes was performed using eggNOG-mapper (version 2.1.9) based on the eggNOG orthology database (version 5.0.2). Sequence searches were performed using DIAMOND (version 2.0.15).

### Taxonomic and Functional Profiles of Samples

Taxonomic profiles at the species and genus levels were obtained using a marker gene-based approach with mOTUs2 (version 3.0.3). For the quantification of gene function, the metagenomic reads were mapped to the non-redundant genes using minimap2 with an identity threshold of 95% or higher, and the number of mapped reads for each gene was counted. The number of multiply mapped reads was distributed to the mapped genes based on the ratio of the number of uniquely mapped reads to genes. The counts were normalized to transcripts per million to form a transcripts per million matrix.

### Physical Parameters

In the population of subjects with an FMD (%) of 4% or more at week 0, the total blood cholesterol level decreased in the test food group (week 8: -2.75 ± 11.51 mg/dL, week 12: -3.75 ± 21.09 mg/dL), and the change from week 0 to week 8 in the test food group was greater than that in the placebo group (3.87 ± 13.74 mg/dL) (*p* = 0.081 by Student's *t*-test). The blood LDL-c level decreased in the test food group (week 8: -4.17 ± 9.18 mg/dL, week 12: -4.29 ± 15.14 mg/dL). There was a statistically significant difference in the change in blood LDL-c level from week 0 to week 8 between the test food group and the placebo group (3.57 ± 10.93 mg/dL) (*p* = 0.012 by Student's *t*-test). The change in blood LDL-c level from week 0 to week 12 in the test food group was greater than that in the placebo group (2.14 ± 9.73 mg/dL) (*p* = 0.092 by Student's *t*-test). The blood PAI-1 level decreased in the test food group (week 8: -1.83 ± 5.25 ng/mL, week 12: -1.25 ± 4.66 ng/mL). The change in blood PAI-1 level from week 0 to week 8 in the test food group was greater than that in the placebo group (0.52 ± 2.61 ng/mL) (*p* = 0.058 by Student's *t*-test), and the change in blood PAI-1 level from week 0 to week 12 in the test food group was greater than that in the placebo group (0.86 ± 3.12 ng/mL) (*p* = 0.076 by Student's *t*-test) (Table 4).

According to the test described above, the effects of intake of yogurt containing *Bifidobacterium animalis* subsp. *lactis* GCL2505 and inulin on vascular endothelial function in healthy adults were studied.

Flow-Mediated Dilatation (FMD) was used in this test. FMD is a vascular endothelial function test that utilizes a blood flow-dependent vasodilatory response that occurs after the release of upper arm occlusion. Increased blood flow after occlusion release causes shear stress on the vascular endothelium, which in turn produces vasodilators, such as nitric oxide. These substances act on vascular smooth muscle adjacent to the vascular endothelium, causing the vascular smooth muscle to relax, which in turn triggers a vasodilatory response. The ratio of the maximally dilated vessel diameter to resting vessel diameter is defined as FMD (%). When vascular endothelial dysfunction occurs, the bioavailability of nitric oxide produced and released from the vascular endothelium decreases, resulting in a reduced vasodilatory response and a low FMD (%). FMD is recognized as noninvasive, low patient-burden, and useful for assessing the risk of developing CVD (NPL 9).

In this test, the test food group showed a 0.85% increase in FMD (%) after consuming the yogurt containing GCL2505 and inulin for 12 weeks. Furthermore, the change in FMD (%) from week 0 to week 12 was significantly greater in the test food group than in the placebo group.

Analysis of the fecal microbiota of the subjects showed no effect of GCL2505 and inulin in alpha-diversity and beta-diversity analyses. It has been reported that dietary fiber intervention in healthy humans increases the abundance of specific bacteria but does not affect the diversity of the intestinal microbiota (NPL 10). The lack of a change in the fecal microbiota may be because the subjects in this study were healthy and did not have intestinal dysbiosis. The relative abundance of *B. animalis* increased only in the intestines of the subjects in the active group and did not increase in the placebo group. In other words, the ingested GCL2505 reached the intestine alive. The increased relative abundance of *B. animalis* in the intestine may have increased the production of acetic acid, which is a major SCFA.

In this test, an effect on blood LDL cholesterol (LDL-c) levels in the test food group was confirmed. LDL-c deposited in the vascular wall is converted into oxidized LDL, and such stimulation contributes to dysfunction of vascular endothelial cells and increased expression of adhesion factors. Oxidized LDL is taken up by macrophages and is involved in the formation of foam cells, thereby maintaining the growth of atherosclerotic lesions by contributing to local and systemic plaque inflammation. Specifically, the improvement in vascular endothelial function observed in this test may have been caused by a decrease in blood LDL-c level. Furthermore, in this test, the blood plasminogen activator inhibitor-1 (PAI-1) level was lower in the test food group than in the placebo group (week 8, *p* = 0.058; week 12, *p* = 0.076). PAI-1 is a cytokine that regulates fibrinolysis and that strongly inhibits thrombolysis by blocking tissue plasminogen activator (t-PA), which is a known thrombolytic agent on fibrin. It has been reported that elevations in PAI-1 levels correlate with atherothrombosis, and that PAI-1 levels were increased in obese metabolic syndrome patients and type II diabetics. Specifically, it can be considered that the reason why intake of GCL2505 and inulin improved vascular endothelial function is that the blood PAI-1 level decreased via the reduction of visceral fat, and fibrinolysis was activated. Furthermore, because it has been reported that there is a positive correlation between visceral fat and blood LDL-c levels, it is possible that the visceral fat mass may have been affected in the test food group, which was confirmed to have reduced blood LDL-c levels, in this test.

It is hypothesized that the improvement of vascular endothelial function by GCL2505 and inulin was achieved by a mechanism of action consisting of the following two steps. Step 1: intake of GCL2505 and inulin exerts a visceral fat-reducing effect via changes in the intestinal microbiota. Clinical studies have clarified that the daily consumption of yogurt containing GCL2505 reduces abdominal visceral fat mass. In addition, Horiuchi et al. demonstrated that administration of GCL2505 increases intestinal *B. lactis* counts and the concentration of acetic acid in plasma, subsequently regulating host energy metabolism in a GPR43-dependent manner (e.g., suppressed body fat accumulation, improved insulin sensitivity, and enhanced whole body fatty acid metabolism) in GPR43 knockout mice. GPR43 is a member of the G protein-coupled receptor family known as short-chain fatty acid receptors in the host, and it has been reported that acetic acid inhibits fat accumulation by activating GPR43 in adipocytes and modulating insulin signaling in adipocytes. Step 2: the development of vascular endothelial dysfunction is prevented through the following sub-steps triggered by a reduction of visceral fat: 1) reduction in blood LDL-c level through a decrease in blood lipid level via a reduction in visceral fat: LDL-c is a causative agent of plaque formation, and its synthesis is related to the amount of free fatty acids released from visceral adipose tissue; and 2) suppression of plasminogen activator inhibitor-1 (PAI-1) expression via a reduction in visceral fat: PAI-1 is an adipocytokine produced by adipocytes and regulates fibrinolysis, which is strongly associated with arteriosclerosis. In this test, it has been proven that GCL2505 and inulin contribute to vascular endothelial function via the inhibitory effect on fat accumulation.

The above results suggest that intake of a synbiotic drink containing GCL2505 and inulin reduces visceral fat, thereby improving vascular endothelial function. Because a meta-analysis reported that a 1% increase in FMD (%) results in an approximately 13% reduction in the risk of developing CVD, the findings of this study are considered clinically significant. Furthermore, since a correlation between FMD (%) and kidney function has also been reported, improving vascular endothelial dysfunction can also possibly reduce the risk of developing kidney disease. Accordingly, because the combination has the potential to reduce the risk of developing not only CVD but also multiple diseases, and to greatly contribute to promoting health, the results obtained this time are considered clinically significant.

## Claims

1. A composition for improving vascular endothelial function, comprising bifidobacteria and water-soluble dietary fiber.

2. The composition according to claim 1, wherein the improvement of vascular endothelial function includes a decrease in blood LDL-C level or a decrease in PAI-1 level, or both.

3. A composition for maintaining or preventing a decline in vascular flexibility or elasticity, comprising bifidobacteria and water-soluble dietary fiber.

4. A composition for preventing arteriosclerosis, comprising bifidobacteria and water-soluble dietary fiber.

5. A composition for improving kidney function, comprising bifidobacteria and water-soluble dietary fiber.

6. The composition according to any one of claims 1 to 5, wherein the water-soluble dietary fiber comprises inulin.

7. The composition according to any one of claims 1 to 5, wherein the bifidobacteria have an ability to proliferate in the gut.

8. The composition according to any one of claims 1 to 5, wherein the bifidobacteria have an anti-metabolic syndrome effect.

9. The composition according to any one of claims 1 to 5, which is a food product.

10. The composition according to any one of claims 1 to 5, further comprising arginine.

11. Use of bifidobacteria and water-soluble dietary fiber for the manufacture of a composition for improving vascular endothelial function.

12. The use according to claim 11, wherein the improvement of vascular endothelial function includes a decrease in blood LDL-C level or a decrease in PAI-1 level, or both.

13. Use of bifidobacteria and water-soluble dietary fiber for the manufacture of a composition for maintaining or preventing a decline in vascular flexibility or elasticity.

14. Use of bifidobacteria and water-soluble dietary fiber for the manufacture of a composition for preventing arteriosclerosis.

15. Use of bifidobacteria and water-soluble dietary fiber for the manufacture of a composition for improving kidney function.

16. Use of water-soluble dietary fiber for the manufacture of a composition for improving vascular endothelial function, the composition comprising bifidobacteria.

17. The use according to claim 16, wherein the improvement of vascular endothelial function includes a decrease in blood LDL-C level or a decrease in PAI-1 level, or both.

18. Use of water-soluble dietary fiber for the manufacture of a composition for maintaining or preventing a decline in vascular flexibility or elasticity, the composition comprising bifidobacteria.

19. Use of water-soluble dietary fiber for the manufacture of a composition for preventing arteriosclerosis, the composition comprising bifidobacteria.

20. Use of water-soluble dietary fiber for the manufacture of a composition for improving kidney function, the composition comprising bifidobacteria.

21. The use according to any one of claims 11 to 20, wherein the water-soluble dietary fiber comprises inulin.

22. The use according to any one of claims 11 to 20, wherein the bifidobacteria have an ability to proliferate in the gut.

23. The use according to any one of claims 11 to 20, wherein the bifidobacteria have an anti-metabolic syndrome effect.

24. The use according to any one of claims 11 to 20, wherein the composition is a food product.

25. The use according to any one of claims 11 to 20, wherein the composition further comprises arginine.
